# EUROPÄISCHE PATENTSCHRIFT

(11) **EP 1 880 017 B1**
(45) Veröffentlichungstag und Bekanntmachung des Hinweises auf die Patenterteilung: **25.08.2010**
(21) Anmeldenummer: 06724612.4
(22) Anmeldetag: 27.04.2006
(51) Int. Cl.: C12Q 1/68

(54) **VERFAHREN ZUR HERSTELLUNG VON LIGANDEN, LIGANDEN UND TEST-KIT**
PROCESS FOR PRODUCING LIGANDS, LIGANDS AND TEST KIT
PROCÉDÉ POUR PRODUIRE DES LIGANDS, LIGANDS ET KIT DE TEST

(30) Priorität: 09.05.2005 DE 102005022057
(43) Veröffentlichungstag der Anmeldung: 23.01.2008
(73) Patentinhaber: Analyticon Biotechnologies AG, 35104 Lichtenfels (DE)
(72) Erfinder: OBERSTRASS, Jürgen, 34317 Habichtswald (DE); MEISEGEIER, Bernhard, 97209 Veitshöchheim (DE); ZWINGMANN, Detlev, 35037 Marburg (DE)
(74) Vertreter: Schneider, Peter Christian
(86) Internationale Anmeldenummer: PCT/EP2006/003927
(87) Internationale Veröffentlichungsnummer: WO 2006/119872

(56) Entgegenhaltungen:
- DE-A1- 10 048 944
- US-A- 5 872 015
- WEINER CHRISTIAN ET AL: "Affinity cross-flow filtration: Purification of IgG with a novel protein a affinity matrix prepared from two-dimensional protein crystals" BIOTECHNOLOGY AND BIOENGINEERING, Bd. 44, Nr. 1, 1994, Seiten 55-65, XP002395192 ISSN: 0006-3592

## Beschreibung

Die Erfindung bezieht sich auf ein Verfahren zur Herstellung von mit einer Zielsubstanz bindungsfähigen Liganden, umfassend die folgenden Schritte:
- In-Kontakt-Bringen der Zielsubstanz mit einem Kandidaten-Ensemble aus einer Mehrzahl unterschiedlicher, vermehrbarer Kandidaten-Liganden, von denen wenigstens einige mit der Zielsubstanz bindungsfähig sind, um ein Binden zwischen den mit der Zielsubstanz bindungsfähigen Kandidaten-Liganden und der Zielsubstanz zu ermöglichen,
- Separieren oder nicht mit der Zielsubstanz bindenden Kandidaten-Liganden von den an die Zielsubstanz gebundenen Kandidaten-Liganden mittels eines physikalischen Trennverfahrens und Verwerfen der separierten Kandidaten-Liganden,
- Lösen der Bindung zwischen den nicht verworfenen Kandidaten-Liganden und der Zielsubstanz und
- Vermehren wenigstens einiger der nicht verworfenen Kandidaten-Liganden.

Die Erfindung bezieht sich weiter auf entsprechend hergestellte Liganden sowie ein diagnostisches Test-Kit, umfassend derartige Liganden.

Ein Verfahren wie oben genannt ist bekannt aus EP 0533838 B1, dessen deutsche Übersetzung als DE 69128350 T2 veröffentlich ist. Die genannte Druckschrift befasst sich mit der Herstellung so genannter Aptamere. Unter einem Aptamer versteht man ein spezifisch bindendes Oligonukleotid, wobei die Zielsubstanz, welche von dem Oligonukleotid spezifisch gebunden wird, keiner grundsätzlichen Beschränkung unterliegt. Es kann sich um eine Nukleinsäuresequenz, ein Protein, ein Zelloberflächenelement oder Ähnliches handeln.

Die Herstellung von Oligonukleotiden mit vorbestimmter Abfolge von Nukleinsäuren ist seit langem bekannt. Die Bindungseigenschaften, insbesondere spezifische Bindungseigenschaften eines synthetisierten Oligonukleotids lassen sich jedoch nur schwer oder gar nicht vorhersagen. Es ist daher üblich, wie in der EP 0533838 B1 beschrieben, erwünschte Aptamere aus einem großen Ensemble von Kandidaten-Oligonukleotiden zu selektionieren. Hierzu bietet man dem Kandidaten-Ensemble die interessierende Zielsubstanz unter Bedingungen an, die eine Bindung grundsätzlich ermöglichen. Anschließend wird die Mischung aus Kandidaten-Liganden und Zielsubstanz einem physikalischen Trennverfahren, beispielsweise einer Gelelektrophorese, unterworfen. Mit diesem Verfahren können ungebundene Liganden von Zielsubstanz-Liganden-Komplexen getrennt werden. Die ungebundenen Oligonukleotide können verworfen werden, da sie nachweislich keine Bindung mit der Zielsubstanz eingehen können, und als spezifische Liganden somit ausscheiden. In einem nachfolgenden Schritt wird die Bindung der Zielsubstanz-Liganden-Komplexe gelöst und die Liganden aufgereinigt und vermehrt. Letzteres erfolgt bei Oligonukleotiden durch übliche und bekannte Amplifikationsverfahren. Zur Verbesserung der Spezifität der aufgefundenen Aptamere wird der beschriebene Prozess bei den bekannten Verfahren mehrfach wiederholt, wobei als Kandidaten-Ensemble, jeweils die zuvor amplifizierten Oligonukleotide verwendet werden.
Das bekannte, üblicherweise als SELEX bezeichnete Verfahren weist den Nachteil auf, dass es sich auch nach vielmaliger Iteration des Verfahrens bei den letztendlich aufgefundenen Liganden stets wieder um ein Ensemble verschiedener Nukleotide handelt. Um "reine" Aptamer-Spezies zu erhalten, ist es daher erforderlich, die Mitglieder des Ensembles zur Vereinzelung zu klonieren und zu sequenzieren und aufgrund von statistischen Erwägungen einen oder mehrere der erhaltenen Klone experimentell auf die erwünschte Bindungsfähigkeit zu überprüfen und im positiven Fall zu weiterzuverwenden. Zwar kann bei diesem Vorgehen mit großer Wahrscheinlichkeit von einer hohen Affinität der Bindung des aufgefundenen Liganden mit der Zielsubstanz ausgegangen werden; seine Spezifität muss jedoch in nachfolgenden Kreuzreaktionstests überprüft werden. Stellt sich bei solchen Tests eine unerwünscht niedrige Selektivität des aufgefundenen Liganden heraus, muss das gesamte Verfahren wiederholt werden, ohne dass der Bediener die Möglichkeit hätte, gezielt auf den oben beschriebenen Selektionierungsprozess einzuwirken, um zu einem besseren Ergebnis, d.h. zu einem echt spezifischen Liganden zu kommen.

DE 100 48 944 A1 offenbart ein Verfahren zur Herstellung von Aptameren, bei dem das Kandidaten-Ensemble zunächst einer Elektrophorese unterworfen wird. Zur weiteren Verwendung wird eine durch einen Laufzeitparameter der Elektrophorese definierte Fraktion isoliert. Diese Fraktion lässt man mit Zielmolekülen reagieren. Bei einer erneuten Elektrophorese unter denselben Bedingungen kann die durch denselben Laufzeitparameter definierte Fraktion verworfen werden, da sie durch die Reaktion mit der Zielsubstanz offensichtlich nicht beeinflusst wurde. D.h. insbesondere nicht mit der Zielsubstanz gebunden hat. Die übrigen Fraktionen, die dagegen eine Beeinflussung durch die Zielsubstanz zeigen, werden sämtlich oder teilweise zu einem neuen Kandidaten-Ensemble zusammengefasst, welches das vorgenannte Verfahren erneut durchläuft. Dieses Verfahren weist zwei wesentliche Nachteile auf. Zum einen ist es durch die vielfache sehr zeitaufwendige Elektrophorese sehr langwierig. Zum anderen erfolgt bereits in der ersten Stufe, d.h. bei der Auswahl einer Fraktion aus dem Kandidaten-Ensemble ein willkürlicher Schritt, bei dem ein Großteil des Kandidaten-Ensembles aus Gründen, die mit ihrer Eignung als spezifische Binder für die Zielsubstanz nichts zu tun haben, verworfen wird.

DE 100 49 074 A1 offenbart ein weiteres Verfahren zur Herstellung von Aptameren, bei dem die Zielsubstanz auf einer Säule immobilisiert ist. Ein Kandidaten-Ensemble wird anschließend über die Säule laufen gelassen, wodurch die Kandidaten-Liganden mit den immobilisierten Zielmolekülen binden und selbst immobilisiert werden können. Der Laufweg der Kandidaten-Liganden auf der Säule wird als Hinweis auf ihre entsprechende Affinität interpretiert. Anschließend wird die Säule isoliert und einzelne Liganden-Fraktionen geordnet nach ihrer Bindungsaffinität, isoliert. Auch dieses Verfahren weist den Nachteil auf, dass es höchst aufwendig gestaltet ist. Zum anderen funktioniert das Verfahren nur für große Mengen von Kandidaten-Liganden, da die angenommene Korrelation zwischen Affinität und Laufweg ein Ausfluss des Massenwirkungsgesetzes ist und somit als statistische Aussage nur bei großen Molekülzahlen zutrifft. Gerade dies ist jedoch für die Bildung reiner Aptamer-Fraktionen hinderlich.

Die US 5,683,916 offenbart ein Verfahren zur Affinitätsreinigung von Kandidaten-Liganden in einem kontinuierlichen Cross-Flow-Filtrationsverfahren, wobei in einer aus Hohlfasern aufgebauten Filtermembran Moleküle der Zielsubstanz immobilisiert sind, die bindungsfähige Liganden in der Membran festhalten, sodass sie durch nachfolgendes Waschen und Eluieren gewonnen werden können. Dieses bekannte Verfahren hat zum einen den Nachteil, dass die Filtermembran durch spezielle Vorbehandlung, insbesondere Immobilisierung der Zielsubstanz für jeden speziellen Anwendungsfall maßgeschneidert werden muss. Dies ist teuer und zeitaufwändig. Zum anderen hat das Verfahren den Nachteil, dass nicht differenziert wird zwischen Kandidaten-Liganden, die zwar die gemeinsame Eigenschaft haben, mit der Zielsubstanz bindungsfähig zu sein, ansonsten jedoch eine unterschiedliche Struktur aufweisen können.

Die US 5,872,015 offenbart ebenfalls ein Verfahren zur Affinitätsreinigung von Kandidaten-Liganden. In einer speziellen Vorrichtung werden ohne Immobilisierung von Zielsubstanzen bindungsfähige Kandidaten-Liganden akkumuliert. Die akkumulierten Kandidaten-Liganden liegen als an die Zielsubstanz gebundene Komplexe vor. Zur Differenzierung zwischen Kandidaten-Liganden hoher und niedriger Affinität zur Zielsubstanz erfolgt ein Eluieren in zwei Schritten mit unterschiedlichen Pufferlösungen. Zur weiteren Differenzierung wird eine Analyse mittels Massenspektroskopie vorgeschlagen. Nachteilig bei diesem Verfahren ist, dass die massenspektroskopische Analyse mit hohem Zeit- und Kostenaufwand verbunden ist. Zudem vermittelt eine solche Analyse lediglich eine Kenntnis der Zusammensetzung des Ensembles ausgewählter Kandidaten-Liganden, erlaubt hingegen keine Verbesserung der Reinheit bzw. Homogenität des Ensembles.

US 5,3891,742 offenbart ein Verfahren zur Bestimmung relativer Affinitäten zweier Komponenten zu einer Zielsubstanz. Dabei wird ein Komponenten-Ensemble mit beiden Komponenten mit der Zielsubstanz in zwei unterschiedlichen Mengenverhältnissen gemischt, wobei aus beiden Mischungen, die jeweils nicht bindende Komponente ausgesondert wird. Anschließend werden beide Mischungen massenspektroskopisch untersucht und die relativen Affinitäten der Komponenten durch Vergleich der Massenspektren bestimmt. Dieses Verfahren ist zur Affinitätsaufreinigung; insbesondere eines heterogenen Kandidaten-Ensembles nicht geeignet.

US 6,180,348 B1 offenbart ein Verfahren zum Identifizieren eines Aptamers. Dabei werden Oligonukleotide unterschiedlicher Sequenzen jeweils auf festen Trägern und gemeinsam auf einem festen Grundträger fixiert. Nachfolgend lässt man Zielsubstanz, die auf magnetischen Kügelchen immobilisiert ist, mit den fixierten Oligonukleotiden reagieren und trennt durch Magnetkraft diejenigen Oligonukleotide von dem Grundträger, deren Wechselwirkung mit der Zielsubstanz stärker als die Wechselwirkung ihres Trägers mit dem Grundträger ist. Wesentlicher Nachteil dieses Verfahrens ist die Notwendigkeit, bereits im Vorfeld dieses Identifizierungsschrittes über reine Oligonukleotide in hinreichend großen Mengen zu verfügen. Dies ist vor allem durch explizite Synthese möglich, was das Auswahlverfahren langwierig und ineffizient macht.

Aus Weiner et al . : "Affinity Cross-Flow Finltration: Purification of IgG with a Novel Protein A Affini ty Matrix Prepared from Two-Dimensional Protein Crystals" Biotechnology and Bioengeneering, Bd. 44, S. 55-65 (1994*)* ist ein Verfahren zur Aufreinigung von IgG mittels Affinitäts-Überstromfiltration bekannt. Dabei werden aus Zellfragmenten von *Clostridium thermohydrosulfuricum L111-69* gewonnene, Protein A enthaltende Mikropartikel zur Affinitätsbindung der zu isolierenden IgG genutzt, um die IgG im Retentat einer Überstromfiltration zu halten bis sämtliche Verunreinigungen aus der Ausgangslösung ausgewaschen sind. Anschließend wird die Bindung zwischen dem FC-Teil der IgG und dem Protein A durch Einsatz eines sauren Elutionspuffers gelöst, sodass die IgG in das Filtrat gelangen und isoliert aufgefangen werden können. Dieses Verfahren stellt ein reines Aufreinigungsverfahren dar. Die eigentliche Herstellung der spezifischen Liganden, nämlich der IgG, muss in einem vorangehenden Schritt erfolgen, in dem insbesondere ihre Spezifität für die eigentliche Zielsubstanz, die regelmäßig nicht das nur zur Aufreinigung verwendete Protein A ist, erzeugt wird.

Es ist die Aufgabe der vorliegenden Erfindung, ein Verfahren zur Herstellung von Liganden zur Verfügung zu stellen, welches die Nachteile des Standes der Technik überwindet und insbesondere geeignet ist, nicht-iterativ zu spezifischen Liganden zu gelangen.

Diese Aufgabe wird in Verbindung mit den Merkmalen des Oberbegriffs von Anspruch 1 dadurch gelöst, dass zur Trennung gebundener und ungebundener Kandidaten-Liganden ein kontinuierliches Cross-Flow-Filtrationsverfahren eingesetzt wird, bei dem eine die Kandidaten-Liganden und die Zielsubstanz enthaltende Filtrationsflüssigkeit mittels einer von der Filtrationsflüssigkeit überströmten Filtereinheit in ein die Zielsubstanz und die daran gebundenen Kandidaten-Liganden enthaltendes Retentat einerseits und ein die ungebundenen Kandidaten-Liganden enthaltendes Filtrat andererseits getrennt wird, wobei das Filtrat aus dem Flüssigkeitsstrom abgeschieden wird,
und weiter dadurch gekennzeichnet, dass nach dem Verwerfen der ungebundenen Kandidaten-Liganden und vor dem Vermehrungsschritt die folgenden Schritte ausgeführt werden:
- Ändern wenigstens eines die Bindung zwischen Kandidaten-Liganden und Zielsubstanz beeinflussenden chemischen und/oder physikalischen Parameters,
- Abscheiden, nach jeder von mehreren Änderungsstufen, derjenigen Kandidaten-Liganden, deren Bindung mit der Zielsubstanz aufgrund der Parameter-Änderung gelöst wurde, in eine der jeweiligen Änderungsstufe zugeordnete Liganden-Fraktion und
- Separates Vermehren wenigstens einiger der gewonnenen Liganden-Fraktionen.

Man beachte, dass das erfindungsgemäße Verfahren grundsätzlich auf jede Art von Liganden anwendbar ist. So kann beispielsweise vorgesehen sein, dass das Kandidaten-Ensemble anstelle von oder zusätzlich zu Oligonukleotiden Nukleinsäure-Protein-Komplexe, arretierte Translationskomplexe, Phagen, Bakterien und/oder eukariontische Zellen umfasst. Zur Vereinfachung der Beschreibung wird die Erfindung jedoch nachfolgend am Beispiel der Oligonukleotide erläutert. Die Übertragung auf andere Ligandentypen kann der Fachmann im Lichte der hiesigen Erläuterungen unschwer durchführen.

Erfindungsgemäß ist vorgesehen, dass die Kandidaten-Liganden und die Zielsubstanz gemeinsam einem Cross-Flow-Filtrationsverfahren, auch bekannt als Überstromfiltration, unterworfen werden. Beim Cross-Flow-Filtrationsverfahren wird ein als Filtrat bezeichneter Anteil der Filtrationsflüssigkeit mittels einer Filtriereinheit, z.B. einer Filtermembran oder eines Hohlfaserbündels oder ähnliches von dem als Retentat bezeichneten Anteil der Filtrationsflüssigkeit, der kontinuierlich über die Filtereinheit zirkuliert, abgetrennt. Strömungsgeschwindigkeit, Filtratvolumen etc. können dabei durch Einstellungen von Geräteparametern, wie etwa Drücke, Pumpengeschwindigkeiten, Porengrößen der Filtriereinheit etc., in dem Fachmann geläufiger Art und Weise und entsprechend den Anforderungen der speziellen Anwendung eingestellt werden. Bei der vorliegenden Erfindung werden die Einstellungen so vorgenommen, dass ungebundene Kandidaten-Liganden mit dem Filtrat abgeschieden werden, wohingegen Zielsubstanz-Liganden-Komplexe, die sich durch Bindung eines Kandidaten-Liganden an ein Zielsubstanzteilchen bilden, im Retentat verleiben. Die derart mit dem Filtrat ausgeschiedenen, ungebundenen Kandidaten-Liganden können verworfen werden, da sie offensichtlich nicht mit der Zielsubstanz bindungsfähig sind und somit als die erwünschten Liganden nicht in Frage kommen.

Wie erläutert, verbleiben im Retentat neben der Zielsubstanz sämtliche Kandidaten-Liganden, welche an die Zielsubstanz gebunden haben. Erfindungsgemäß ist nun ein weiterer Schritt vorgesehen, bei dem wenigstens ein chemischer und/oder physikalischer Parameter, der die Bindung zwischen den Kandidaten-Liganden und der Zielsubstanz beeinflusst, geändert wird. Die Änderung kann langsam kontinuierlich oder - vorzugsweise - stufenweise erfolgen, wobei die Stufenhöhe und Änderungsrichtung so zu bemessen sind, dass ein Teil der Zielsubstanz-Ligandenkomplexe gelöst wird, so dass freie Liganden und freie Zielsubstanz-Teilchen entstehen. Im Rahmen der nach wie vor laufenden Cross-Flow-Filtration gelangen die frei gewordenen Kandidaten-Liganden in das Filtrat, welches im Gegensatz zum oben erläuterten ersten Verfahrensschritt jedoch nicht verworfen, sondern in einer der aktuellen Parameter-Änderungsstufe zugeordneten Ligandenfraktion aufgefangen wird. Man beachte, dass der Begriff der Änderungsstufe bzw. Stufenhöhe im Fall einer kontinuierlichen Parameteränderung einen vorgegebenen Parameterbereich bezeichnet, wobei alle Kandidaten-Liganden, die während eines Überstreichens dieses Bereichs ihre Bindung mit der Zielsubstanz gelöst haben, in einer entsprechenden gemeinsamen Fraktion aufgefangen werden.

Dieser Fraktionierungsschritt, d.h. das Ändern eines die Bindung beeinflussenden Parameters und das nachfolgende Auffangen der abgelösten Liganden in einer entsprechenden Fraktion wird mehrfach wiederholt, um zu einer Mehrzahl von Liganden-Fraktionen zu gelangen, die unterschiedliche Bindungseigenschaften in Bezug auf die Zielsubstanz aufweisen, was sich insbesondere in den unterschiedlichen Lösungsbedingungen der Bindung, die durch die unterschiedlichen Fraktionen repräsentiert werden, verdeutlicht. Es kann daher mit großer Wahrscheinlichkeit angenommen werden, dass die in verschiedenen Fraktionen gesammelten Liganden mit verschiedenen Bindungseigenschaften auch strukturell, d.h. im Fall von Oligonukleotiden z.B. in ihrer Sequenz, verschieden sind. Bei hinreichend feiner Abstufung der Parameteränderung ist es somit im Gegensatz zum Stand der Technik möglich, nahezu reine Fraktionen von Kandidaten-Liganden zu gewinnen, die alle grundsätzlich mit der Zielsubstanz bindungsfähig sind. Je nach geplanter Verwendung kann der zu ändernde Parameter eine Temperatur, ein ph-Wert oder ein Salzgehalt der Filtrationsflüssigkeit sein. Auch Konzentrationen von Detergenzien, chaotropen Agenzien, denaturierenden Substanzen (z.B. Formamid oder Harnstoff) oder kompetitiven Liganden können als Parameter variiert werden.

In einem abschließenden Schritt können diese Fraktionen oder zumindest einige davon separat amplifiziert werden und ihre Bindungseigenschaften im Hinblick auf ihre Spezifität zur Zielsubstanz in Kreuzreaktions- oder anderen Tests genauer untersucht werden. Führt dann ein solcher Test zu dem Ergebnis, dass die speziell untersuchte Liganden-Fraktion, die für eine spezielle Anwendung erforderlichen Bedingungen nicht erfüllt, muss nicht, wie im Stand der Technik, das gesamte Verfahren wiederholt werden, sondern es reicht aus, eine weitere, bereits gewonnene und amplifizierte Fraktion demselben Test zu unterwerfen. Auf diese Weise wird zum einen weitestgehend sichergestellt, dass sämtliche in dem ursprünglichen Ensemble enthaltenen Kandidaten-Liganden einer angemessenen Tauglichkeitsprüfung unterzogen werden. Beim Stand der Technik ist dies nicht der Fall, da der Prozess dort im wesentlichen zufälligen Selektionsmechanismen unterworfen ist, die dazu führen können, dass auch bei vielmaliger Wiederholung des gesamten Verfahrens bestimmte Kandidaten-Liganden niemals "Sieger" des zufälligen Selektionsprozesses bleiben und daher niemals für konkrete Anwendungstests in Betracht gezogen werden. Zum anderen beschleunigt das erfindungsgemäße Verfahren die Produktion der erwünschen Liganden wesentlich, da in einem einzigen Verfahrensdurchlauf eine Vielzahl von grundsätzlich tauglichen Liganden-Fraktionen entstehen.

Ein weiterer Vorteil des erfindungsgemäßen Verfahrens ist es, dass von jeder einzelnen Liganden-Fraktion genau diejenigen Bedingungen bekannt sind, bei denen die Bindung mit der Zielsubstanz gelöst wurde. Diese physiko-chemische Zusatzinformation kann vom Fachmann genutzt werden, um bestimmte Liganden-Fraktionen für bestimmte Anwendungen besonders in Betracht zu ziehen oder auszuschließen. Auch dies dient einer zielgerichteteren Entwicklung spezieller Wirksubstanzen.

Im Rahmen des Cross-Flow-Filtrationsverfahrens wird bei einer bevorzugten Ausführungsform der Erfindung der Filtrationsflüssigkeit kontinuierlich oder schrittweise ein dem abgeschiedenen Filtrat entsprechendes von Zielsubstanz und Kandidaten-Liganden freies Flüssigkeitsvolumen zugeführt. Dies bedeutet mit anderen Worten, dass das abgeschiedene Filtratvolumen im Cross-Flow-Kreislauf ersetzt wird. Dies führt zum einen dazu, dass das erfindungsgemäße Verfahren für eine nahezu unbeschränkte Dauer laufen kann, was eine nahezu unbeschränkt feine Abstufung der sich ergebenden Fraktionen ermöglicht. Andererseits führt der Volumenersatz auch zu einer starken Verdünnung der Filtrationsflüssigkeit, da das zugesetzte Flüssigkeitsvolumen keine Kandidaten-Liganden oder Zielsubstanz enthält. Diese Verdünnung führt zu einer deutlichen Verbesserung der Statistik im Hinblick auf reine Fraktionen. Bei einer typischen Anwendung des erfindungsgemäßen Verfahrens für die Herstellung von Aptameren enthält das Kandidaten-Ensemble ca. 10¹¹ verschiedene Oligonukleotide. Von diesen binden jedoch beispielsweise nur ca. 10³ mit der Zielsubstanz. Die Übrigen sollten bereits im ersten Verfahrensschritt als grundsätzlich untauglich verworfen werden. Es ist offensichtlich, dass dieses Ziel allein aufgrund der ungünstigen Statistik nur in den seltensten Fällen erreicht werden wird. Vielmehr muss damit gerechnet werden, dass einige nicht bindende Kandidaten-Liganden im Retentat verbleiben. Wird jedoch das Retentat in den nachfolgenden Schritten durch die vorgenannte Volumenauffüllung stetig weiter verdünnt, im Idealfall soweit, dass die einzelnen Fraktionen Einzel-Ligandenmoleküle enthalten, kann dieser statistische Nachteil überwunden werden, so dass man letztendlich doch reine oder nahezu reine Fraktionen erhält.

Im Rahmen des Vermehrungsschrittes der einzelnen Fraktionen ist bei einer bevorzugten Ausführungsform der Erfindung vorgesehen, dass die vermehrten Liganden mit einer Markierung versehen werden. Dies kann beispielsweise eine Partikelmarkierung, etwa in Form von Gold oder Latexkügelchen sein, eine Fluoreszenzmarkierung, eine enzymatische Markierung oder Ähnliches. Auf diese Weise können die amplifizierten Liganden der einzelnen Fraktionen in einem Bindungstest, der beispielsweise auf einem Mikro-Array oder einer Multiwell-Platte mit immobilisierter Zielsubstanz und/oder konkurrierenden Bindungssubstanzen leicht identifiziert werden.

Bei der konkreten Anwendung des erfindungsgemäßen Verfahrens zur Herstellung von Aptameren, umfasst das Kandidaten-Ensemble, wie bereits erläutert, Oligonukleotide. Vorzugsweise verwendet man hierzu solche Nukleotide, die jeweils einen oder mehrere variable Bereiche aus zufallsveränderten Nukleotidsequenzen aufweisen, die 5'- und 3'-seitig von konstanten Bereichen begrenzt sind.

Dabei umfassen die variablen Bereiche vorzugsweise mindestens 12-30 Nukleotide. Die konstanten Bereiche umfassen vorzugsweise jeweils 12-22 Nukleotide. Es wird bevorzugt, dass die variablen Bereiche jeweils zusammenhängend ausgebildet sind. Es hat sich jedoch ebenfalls als günstig herausgestellt, variable Bereiche zu verwenden, die ein- oder mehrfach von konstanten Bereichen unterbrochen sind, die ihrerseits je 2-5 Nukleotide umfassen. Durch diese Blöcke definierter Sequenz wird erreicht, dass in der Kandidatenmischung Aptamere mit bestimmten Sekundärstrukturen bevorzugt auftreten. So wird z.B. die Ausbildung von helikalen Bereichen bevorzugt, wenn eine Reihe von Cytosinen eingeführt wird, oder die Selektion von Haarnadelstrukturen, wenn der konstante Block eine "Tetraloop-Sequenz" enthält.

Zur praktischen Durchführung des Cross-Flow-Verfahrens im Fall der Herstellung von Aptameren, die beispielsweise an Proteine oder Zellen binden, ist es günstig eine Filtereinheit zu verwenden, deren Porengröße die in der Regel deutlich größeren Proteine bzw. Zellen zurückhält, während die deutlich kleineren Oligonukleotide in ungebundener Form das Filterelement durchdringen und ins Filtrat gelangen können. In anderen Fällen jedoch, in denen die Liganden und die Zielsubstanzteilchen von vergleichbarer Größe sind, hat es sich als günstig erwiesen, die Zielsubstanz an im Vergleich zu einer Porengröße der Filtereinheit große Substratteilchen zu binden. Dies können beispielsweise Gold-, Sepharose-, Agarose-, Latex- oder Glaskügelchen sein, die die Filtereinheit nicht durchdringen können. Es sei jedoch darauf hingewiesen, dass hierdurch keine Immobilisierung der Zielsubstanz an der Filtereinheit erfolgt. Vielmehr strömen die Substratteilchen mit den daran gebundenen Zielsubstanzteilchen frei im Retentat.

Aus der angehenden Erläuterung ergibt sich, dass erfindungsgemäß hergestellte Liganden zum einen deutlich preiswerter sein können als solche nach dem Stand der Technik und außerdem zielgerichteter im Hinblick auf spezielle Anwendungen hergestellt werden können, so dass ihre Optimierung für eine spezielle Anwendung deutlich über das bisher bekannte Maß hinausgehen kann.

Entsprechend sind von diagnostischen Test-Kits, die die erfindungsgemäßen Liganden enthalten, günstigere Preise und bessere Leistung zu erwarten als bislang möglich.

Es sei jedoch darauf hingewiesen, dass sich die Anwendungsmöglichkeiten der erfindungsgemäßen Liganden nicht auf diagnostische in-vitro-Tests beschränken. Vielmehr können die Liganden in jeglichem bindungsabhängigen Nachweisverfahren, z.B. auch im Lebensmittel- oder Umweltbereich oder in der Phytopathologie, eingesetzt werden. Es sind auch therapeutische Anwendungen und Diagnoseverfahren am lebenden Organismus denkbar.

Weitere Vorteile der vorliegenden Erfindung ergeben sich aus der nachfolgenden speziellen Beschreibung sowie der Zeichnung:
Es zeigt:

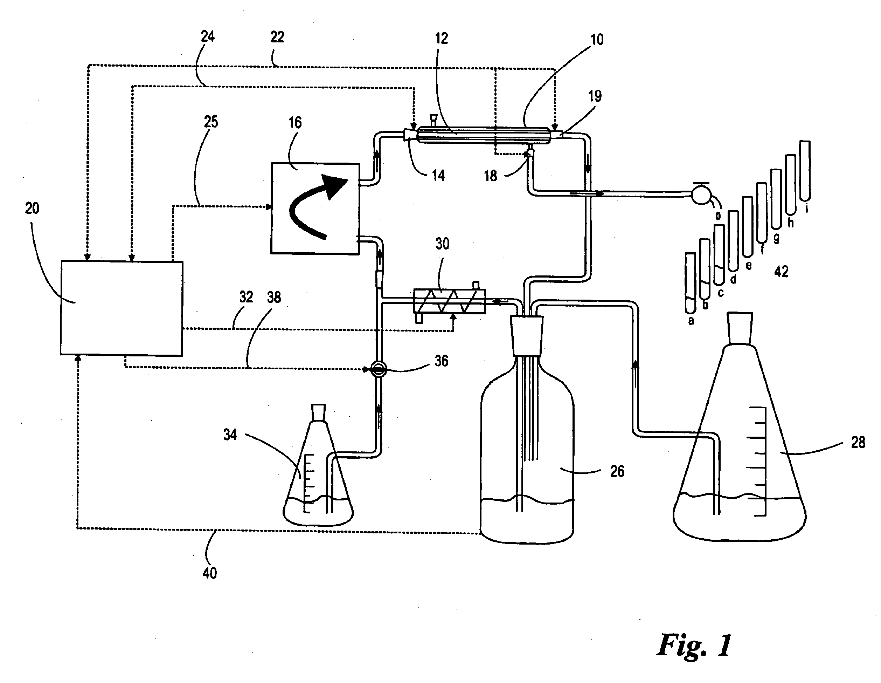

- Figur 1:: eine schematische Darstellung einer Vorrichtung zur Durchführung des erfindungsgemäßen Verfahrens.

Figur 1 zeigt eine schematische Darstellung einer Apparatur zur Durchführung des erfindungsgemäßen Verfahrens, anhand derer der Verfahrensablauf erläutert werden soll. Kernstück der Vorrichtung ist eine Cross-Flow-Filtrationseinheit 10, die auf herkömmliche Weise mit einer Filtermembran oder Filterkartusche 12 ausgestattet ist. Die Cross-Flow-Einrichtung weist einen Eingang 14 auf, über den Filtrationsflüssigkeit mittels einer Pumpe zugeführt wird. Die Cross-Flow-Einheit 10 weist zwei Ausgänge 18 und 19 auf, wobei der Filtratausgang 18 der Ableitung desjenigen Anteils der Filtrationsflüssigkeit dient, der die Filterkartusche 12 passiert hat. Der Retentatausgang 19 hingegen leitet denjenigen Anteil der Filtrationsflüssigkeit ab, der die Filterkartusche 12 nicht passieren konnte. Die relativen Volumina von Filtrat und Retentat sind abhängig von verschiedenen Einstellungsparametern der Cross-Flow-Einrichtung 10. Hierzu gehört die Leistung der Pumpe 16, die Beschaffenheit der Filterkartusche 12 und die Druckverhältnisse an den Ein- und Ausgängen 14, 18, 19. Zur geeigneten Einstellung bzw. Regelung der Parameter ist eine Steuereinrichtung 20 vorgesehen, die über Mess- und Steuerleitungen mit der Pumpe 16 und Druckeinstellvorrichtungen (nicht dargestellt) an der Cross-Flow-Einrichtung 10 verbunden ist. Die Mess- und Steuerleitungen sind in Figur 1 als gestrichelte Pfeile dargestellt, wobei die Pfeilspitzen die vorherrschende Informationsflussrichtung andeuten. So tauscht die Mess- und Steuerleitung 22 Mess- und Steuerdaten mit Druckregelungseinrichtungen an den Ausgängen 18 und 19 aus, die Mess- und Steuerleitung 24 tauscht Mess- und Steuerdaten mit einer Druckeinstellungsvorrichtung am Eingang 14 aus und die Steuerleitung 25 sendet Steuersignale an die Pumpe 16.

Der Eingang der Pumpe 16 ist mit einem Filtrationsflüssigkeitsreservoir 26 verbunden, in welches auch das Retentat vom Ausgang 19 abfließt.

Bei Verfahrensbeginn wird das Reservoir 26 mit Filtrationsflüssigkeit gefüllt, der sowohl das Kandidaten-Ensemble als auch die Zielsubstanz zugesetzt sind. In diesem ersten Verfahrensschritt läuft eine erste Bindungsreaktion zwischen der Zielsubstanz und denjenigen Kandidaten-Liganden ab, die grundsätzlich mit der Zielsubstanz bindungsfähig sind. Die Filtrationsflüssigkeit enthält somit Zielsubstanz-Liganden-Komplexe, ungebundene Liganden und, in Abhängigkeit von den eingesetzten Startmengen, auch ungebundene Zielsubstanz.

Dieses Gemisch wird mittels der Pumpe 16 in Zirkulation durch die Cross-Flow-Einrichtung 10 versetzt. Die Filterkartusche 12 ist dabei so ausgestaltet, dass ungebundene Liganden den Filter passieren können und zum Filtratausgang 18 gelangen, während Zielsubstanz-Liganden-Komplexe und ggf. ungebundene Zielsubstanz im Retentat verbleiben und über den Retentatausgang 19 dem Filtrationsflüssigkeitsreservoir 26 wieder zugeführt werden. In diesem anfänglichen Filtrationsschritt kann das Filtrat vollständig verworfen werden, da die darin enthaltenen Kandidaten-Liganden offensichtlich nicht mit der Zielsubstanz bindungsfähig sind.

Bei der in Figur 1 dargestellten, besonders vorteilhaften, Apparatur ist ein weiteres Flüssigkeitsreservoir 28 vorgesehen, in welchem Filtrationsflüssigkeit ohne Kandidaten-Liganden oder Zielsubstanz vorgehalten wird. Mit dieser Flüssigkeit aus dem Reservoir 28 wird der Volumenverlust durch die Abzweigung des Filtrats ersetzt. Außerdem "verdünnt" sich die zirkulierende Mischung entsprechend, um die Statistik des Vorgangs positiv zu beeinflussen.

Nachdem die ungebundenen Kandidaten-Liganden im Wesentlichen aus der Mischung entfernt sind, wird in einem Folgeschritt ein die Bindung zwischen den Kandidaten-Liganden und der Zielsubstanz beeinflussender Parameter variiert. Der Parameter kann beispielsweise die Temperatur der zirkulierenden Flüssigkeit sein. Dies ist in Figur 1 schematisch durch eine Temperierschlange 30 dargestellt, wobei die Temperaturregelung durch die Steuereinheit 20 über die Mess- und Steuerleitung 32 vorgenommen wird. Alternativ oder zusätzlich kann der zu variierende Parameter chemische Bedingungen der zirkulierenden Flüssigkeit betreffen. Beispielhaft seien hierzu der ph-Wert, Harnstoffkonzentration oder spezielle Salzkonzentrationen genannt. In Figur 1 ist dies durch das Chemikalien-Reservoir 34 dargestellt, aus welchem eine Titrierlösung über das Ventil 36 der zirkulierenden Mischung zutitriert werden kann. Die Steuerung des Ventils 36 erfolgt durch die Steuereinheit 20 über die Steuerleitung 38. In Figur 1 ist eine allgemeine Messleitung 40 zwischen dem Reservoir 26 und der Steuereinheit 20 dargestellt, die schematisch die Messung aller relevanten Parameter sowie die entsprechenden Sensoren darstellen soll. Selbstverständlich wird der Fachmann die geeigneten Sensoren an den geeigneten Stellen der Vorrichtung anbringen und ggf. mehrere Messleitungen kombinieren. Figur 1 ist daher lediglich symbolisch zur Veranschaulichung und nicht als konkreter Bauplan einer entsprechenden Vorrichtung zu verstehen.

Durch die Änderung eines Parameters, wie oben beschrieben, ändern sich die Bindungsbedingungen der Zielsubstanz-Liganden-Komplexe mit der Folge, dass einige Komplexe ihre Bindung lösen werden. Die freiwerdenden Liganden können dann die Filterkartusche 12 passieren und gelangen in das Filtrat, welches in diesem Schritt des Verfahrens nicht mehr verworfen, sondern in einzelnen Fraktionen, die in Figur 1 mit den Bezugszeichen 42 a-i versehen sind, aufgefangen wird. Nachfolgend wird derselbe oder ein anderer Parameter weiter variiert und das sich ergebende Filtrat in einer neuen Fraktion aufgefangen. Dieser Schritt kann prinzipiell beliebig oft wiederholt werden, je nachdem wie viele Paramatervariationsstufen gewünscht sind. Das sich nach jeder Variationsstufe ergebende Filtrat wird in einer eigenen Fraktion aufgefangen, so dass sich im Ergebnis eine Mehrzahl von Fraktionen 42 a-i ergibt, die jeweils einer Parameteränderungsstufe zuordenbar sind. Es kann, wie bereits erläutert, auch eine kontinuierliche Variation der Parameter durchgeführt werden, was dazu führt, dass jeder Fraktion ein Parameterbereich zuzuordnen ist.

Anschließend können wenigstens einige der gewonnenen Fraktionen 42 a-i vermehrt und weiteren Tests bezüglich ihrer Eignung zu speziellen, gewünschten Anwendungen unterzogen werden. Wie bereits im Rahmen der allgemeinen Beschreibung erläutert, kann die Vermehrung auch eine Markierung der in den Fraktionen gewonnenen Liganden und die Tauglichkeitstests Bindungs- und Kreuzreaktionstests umfassen.
Natürlich stellt das in der Zeichnung und der speziellen Beschreibung erläuterte Ausführungsbeispiel nur eine exemplarische und illustrative Ausführungsform der vorliegenden Erfindung dar. Insbesondere der apparative Aufbau kann vom Fachmann im Rahmen der vorliegenden Erfindung vielfältig variiert werden. Dies trifft insbesondere auf eine Anpassung an spezielle Liganden zu, die bevorzugt, jedoch nicht notwendig Oligonukleotide sind, so dass die letztlich gewonnenen Oligonukleotid-Liganden als Aptamere bezeichnet werden können.

## Patentansprüche

1. Verfahren zur Herstellung von mit einer Zielsubstanz bindungsfähigen Liganden, umfassend die folgenden Schritte:
- In-Kontakt-Bringen der Zielsubstanz mit einem Kandidaten-Ensemble aus einer Mehrzahl unterschiedlicher, vermehrbarer Kandidaten-Liganden, von denen wenigstens einige mit der Zielsubstanz bindungsfähig sind, um ein Binden zwischen den mit der Zielsubstanz bindungsfähigen Kandidaten-Liganden und der Zielsubstanz zu ermöglichen,
- Separieren der nicht mit der Zielsubstanz bindenden Kandidaten-Liganden von den an die Zielsubstanz gebundenen Kandidaten-Liganden mittels eines physikalischen Trennverfahrens und Verwerfen der separierten Kandidaten-Liganden,
- Lösen der Bindung zwischen den nicht verworfenen Kandidaten-Liganden und der Zielsubstanz und
- Vermehren wenigstens einiger der nicht verworfenen Kandidaten-Liganden,
**dadurch gekennzeichnet,**
**dass** zur Trennung gebundener und ungebundener Kandidaten-Liganden ein kontinuierliches Cross-Flow-Filtrationsverfahren eingesetzt wird, bei dem eine die Kandidaten-Liganden und die Zielsubstanz enthaltende Filtrationsflüssigkeit mittels einer von der Filtrationsflüssigkeit überströmten Filtereinheit (12) in ein die Zielsubstanz und die daran gebundenen Kandidaten-Liganden enthaltendes Retentat einerseits und ein die ungebundenen Kandidaten-Liganden enthaltendes Filtrat andererseits getrennt wird, wobei das Filtrat aus dem Flüssigkeitsstrom abgeschieden wird,
und weiter **dadurch gekennzeichnet,**
**dass** nach dem Verwerfen der ungebundenen Kandidaten-Liganden und vor dem Vermehrungsschritt die folgenden Schritte ausgeführt werden:
- Ändern wenigstens eines die Bindung zwischen Kandidaten-Liganden und Zielsubstanz beeinflussenden chemischen und/oder physikalischen Parameters,
- Abscheiden, nach jeder von mehreren Änderungsstufen, derjenigen Kandidaten-Liganden, deren Bindung mit der Zielsubstanz aufgrund der Parameter-Änderung gelöst wurde, in eine der jeweiligen Änderungsstufe zugeordnete Liganden-Fraktion (42 a-i) und
- Separates Vermehren wenigstens einiger der gewonnenen Liganden-Fraktionen (42 a-i).

2. Verfahren gemäß Anspruch 1,
**dadurch gekennzeichnet,**
**dass** die Parameteränderung stufenweise erfolgt.

3. Verfahren nach einem der vorangehenden Ansprüche,
**dadurch gekennzeichnet,**
**dass** der veränderte Parameter die Temperatur, der pH-Wert, der Gehalt an denaturierenden Substanzen, ein
Salzgehalt oder die Anwesenheit eines kompetitiven Binders in der Flüssigkeit ist.

4. Verfahren nach einem der vorangehenden Ansprüche,
**dadurch gekennzeichnet,**
**dass** der Filtrationsflüssigkeit kontinuierlich oder schrittweise ein dem abgeschiedenen Filtrat entsprechendes, von Zielsubstanz und Kandidaten-Liganden freies Flüssigkeitsvolumen zugeführt wird.

5. Verfahren nach einem der vorangehenden Ansprüche,
**dadurch gekennzeichnet,**
**dass** die Kandidaten-Liganden während des Vermehrungsschrittes mit einer Markierung verbunden werden.

6. Verfahren nach einem der vorangehenden Ansprüche,
**dadurch gekennzeichnet,**
**dass** das Kandidaten-Ensemble Oligonukleotide unterschiedlicher Sequenzen umfasst.

7. Verfahren gemäß Anspruch 6,
**dadurch gekennzeichnet,**
**dass** das Kandidaten-Ensemble Oligonukleotide umfasst, die jeweils einen oder mehrere variable Bereiche aus zufallsveränderten Nukleotidsequenzen aufweisen, die 5'- und 3'-seitig von konstanten Bereichen begrenzt sind.

8. Verfahren gemäß Anspruch 7,
**dadurch gekennzeichnet,**
**dass** die variablen Bereiche mindesten 12 bis 30 Nukleotide umfassen.

9. Verfahren gemäß Anspruch 7 oder 8,
**dadurch gekennzeichnet,**
**dass** die konstanten Bereiche jeweils 12 bis 22 Nukleotide umfassen.

10. Verfahren gemäß Anspruch 7 oder 9,
**dadurch gekennzeichnet,**
**dass** die variablen Bereiche jeweils zusammenhängend ausgebildet sind.

11. Verfahren gemäß Anspruch 7 oder 9,
**dadurch gekennzeichnet,**
**dass** die variablen Bereiche ein- oder mehrfach von konstanten Bereichen unterbrochen sind, die jeweils 2 bis 5 Nukleotide umfassen.

12. Verfahren nach einem der vorangehenden Ansprüche,
**dadurch gekennzeichnet,**
**dass** das Kandidaten-Ensemble Nukleinsäure-Protein-Komplexe, arretierte Translationskomplexe, Phagen, Bakterien oder eukaryontische Zellen umfasst.

13. Verfahren nach einem der vorangehenden Ansprüche,
**dadurch gekennzeichnet,**
**dass** die Zielsubstanz an im Vergleich zu einer Porengröße der Filtereinheit große Substratteilchen gebunden ist.

## Claims

1. A method for producing ligands capable of binding with a target substance, comprising the following steps:
- Bringing the target substance into contact with a candidate set consisting of a plurality of different, amplifiable candidate ligands at least some of which are capable of binding with the target substance, in order to permit binding between the candidate ligands capable of binding with the target substance and the target substance,
- Separation of the candidate ligands that do not bind to the target substance, from the candidate ligands that are bonded to the target substance, by using a physical separation process, and discarding the separated candidate ligands,
- Breaking the bond between the non-discarded candidate ligands and the target substance and
- Amplifying at least one of the non-discarded candidate ligands,
**characterized in that**
in order to separate the bonded and the unbonded candidate ligands, a continuous cross-flow filtration process is used in which a filtration liquid containing the candidate ligands and the target substance is separated, by means of a filter unit (12) over which the filtration liquid flows, into a retentate that contains the target substance and the candidate ligands bonded thereto, on the one hand, and into a filtrate containing the unbonded candidate ligands, on the other hand, the filtrate being separated out of the liquid flow,
further **characterized in that**,
after the unbonded candidate ligands have been discarded and before the multiplication step, the following steps are carried out:
- Modification of at least one chemical and/or physical parameter that influences the binding between candidate ligands and target substance,
- Separation, after each of several modification steps, of those candidate ligands whose bond with the target substance was dissolved by the parameter modification, into a ligand fraction (42 a-i) associated with the respective modification step and
- Separate multiplication of at least some of the ligand fractions (42 a-i) obtained.

2. A method according to Claim 1,
**characterized in that** the parameter modification takes place in steps.

3. A method according to one of the preceding claims,
**characterized in that** the parameter modified is the temperature, the pH value, the content of denatured substances, a salt content or the presence of a competitive binding agent in the liquid.

4. A method according to any of the preceding claims,
**characterized in that** an amount of liquid free of target substance and candidate ligands that is equivalent to the separated-out filtrate is supplied continuously or gradually to the filtration liquid.

5. A method according to any of the preceding claims,
**characterized in that** the candidate ligands are combined with a marker during the multiplication step.

6. A method according to any of the preceding claims,
**characterized in that** the candidate set comprises oligonucleotides of various sequences.

7. A method according to Claim 6
**characterized in that** the candidate set comprises oligonucleotides that have one or more variable regions consisting of randomly modified nucleotide sequences which are bounded by constant regions on the 5' and the 3' sides.

8. A method according to Claim 7
**characterized in that** the variable regions comprise at least 12 to 30 nucleotides.

9. A method according to Claim 7 or 8,
**characterized in that** each of the constant regions comprises 12 to 22 nucleotides.

10. A method according to Claim 7 or 9,
**characterized in that** each of the variable regions has a continuous structure.

11. A method according to Claim 7 or 9
**characterized in that** the variable regions are interrupted once or several times by constant regions that each comprise 2 to 5 nucleotides.

12. A method according to any of the preceding claims,
**characterized in that** the candidate set comprises nucleic acid protein complexes, arrested translation complexes, phages, bacteria or eukaryotic cells.

13. A method according to any of the preceding claims,
**characterized in that** the target substance is bonded to substrate particles that are large compared with the pore size of the filter unit.

## Revendications

1. Procédé pour produire des ligands pouvant se lier avec une substance cible, comprenant les étapes suivantes :
- mettre en contact la substance cible avec un ensemble de candidats constitué d'une pluralité de ligands candidats différents multipliables, dont au moins certains peuvent se lier avec la substance cible, afin de permettre une liaison entre la substance cible et les ligands candidats pouvant se lier avec la substance cible,
- séparer, des ligands candidats liés à la substance cible, les ligands candidats ne se liant pas avec la substance cible, au moyen d'un procédé de séparation physique, et éliminer les ligands candidats séparés,
- supprimer la liaison entre les ligands candidats non éliminés et la substance cible, et
- multiplier au moins certains des ligands candidats non mis au rebut,
**caractérisé en ce que**,
pour la séparation entre les ligands candidats liés et non liés, on utilise un procédé continu de filtration à courant transversal, selon lequel un liquide de filtration contenant les ligands candidats et la substance cible est séparé, au moyen d'une unité de filtration (12) submergée par le liquide de filtration, en un rétentat contenant la substance cible et les ligands candidats liés à celle-ci d'une part, et un filtrat contenant les ligands candidats non liés d'autre part, sachant que le filtrat est éliminé du flux de liquide,
et **caractérisé en outre en ce que**, après l'élimination des ligands candidats non liés et avant l'étape de multiplication, on effectue les étapes suivantes :
- modification d'au moins un paramètre chimique et/ou physique influant sur la liaison entre les ligands candidats et la substance cible,
- séparation, après chacune de plusieurs étapes de modification, des ligands candidats dont la liaison avec la substance cible a été supprimée en raison de la modification de paramètre, en une fraction de ligands (42 a-i) associée à l'étape de modification respective, et
- multiplication séparée d'au moins certaines des fractions de ligands obtenues (42 a-i).

2. Procédé selon la revendication 1, **caractérisé en ce que** la modification de paramètre s'effectue par étapes.

3. Procédé selon l'une des revendications précédentes, **caractérisé en ce que** le paramètre modifié est la température, le pH, la teneur en substances dénaturantes, une teneur en sel ou la présence d'un liant concurrent dans le liquide.

4. Procédé selon l'une des revendications précédentes, **caractérisé en ce que** le liquide de filtration est apporté en continu ou par étapes à un volume de liquide exempt de substance cible et de ligands candidats correspondant au filtrat extrait.

5. Procédé selon l'une des revendications précédentes, **caractérisé en ce que** les ligands candidats sont combinés, pendant l'étape de multiplication, à un marqueur.

6. Procédé selon l'une des revendications précédentes, **caractérisé en ce que** l'ensemble de candidats comprend des oligonucléotides de différentes séquences.

7. Procédé selon la revendication 6, **caractérisé en ce que** l'ensemble de candidats comprend des oligonucléotides qui présentent respectivement une ou plusieurs régions variables constituées de séquences de nucléotides aléatoirement modifiées, qui sont délimitées sur les côtés 5' et 3' par des régions constantes.

8. Procédé selon la revendication 7, **caractérisé en ce que** les régions variables comprennent au moins 12 à 30 nucléotides.

9. Procédé selon la revendication 7 ou 8, **caractérisé en ce que** les régions constantes comprennent respectivement 12 à 22 nucléotides.

10. Procédé selon la revendication 7 ou 9, **caractérisé en ce que** les régions variables sont respectivement réalisées continues.

11. Procédé selon la revendication 7 ou 9, **caractérisé en ce que** les régions variables sont interrompues une ou plusieurs fois par des régions constantes qui comprennent respectivement 2 à 5 nucléotides.

12. Procédé selon l'une des revendications précédentes, **caractérisé en ce que** l'ensemble de candidats comprend des complexes d'acide nucléique et de protéine, des complexes de translation bloqués, des phages, des bactéries ou des cellules eucaryotes.

13. Procédé selon l'une des revendications précédentes, **caractérisé en ce que** la substance cible est liée à des particules de substrat qui sont grandes relativement à la taille des pores de l'unité de filtration.
